# EUROPÄISCHE PATENTSCHRIFT

(11) **EP 1 971 256 B1**
(45) Veröffentlichungstag und Bekanntmachung des Hinweises auf die Patenterteilung: **27.01.2010**
(21) Anmeldenummer: 06817762.5
(22) Anmeldetag: 18.12.2006
(51) Int. Cl.: A61B 5/00, A61M 5/158, A61M 5/142, A61L 24/00

(54) **MEDIZINISCHE VORRICHTUNG ZUR PLATZIERUNG AUF DER HAUT EINES PATIENTEN**
MEDICAL DEVICE FOR PLACING ON A PATIENT S SKIN
DISPOSITIF MÉDICAL DESTINÉ À ÊTRE PLACÉ SUR LA PEAU D'UN PATIENT

(30) Priorität: 10.01.2006 CH 35062006
(43) Veröffentlichungstag der Anmeldung: 24.09.2008
(73) Patentinhaber: F. Hoffmann-La Roche AG, 4070 Basel (CH); Roche Diagnostics GmbH, 68305 Mannheim (DE)
(72) Erfinder: STETTLER, Marianne, 8302 Kloten (CH); HUNN, Marcel, 4900 Langenthal (CH); WALTER, Christoph, 4552 Derendingen (CH)
(74) Vertreter: Schalch, Rainer
(86) Internationale Anmeldenummer: PCT/CH2006/000711
(87) Internationale Veröffentlichungsnummer: WO 2007/079598

(56) Entgegenhaltungen:
- EP-A- 0 780 138
- EP-A1- 1 213 037
- WO-A-93/09713
- WO-A-97/41917
- WO-A-99/58190
- US-A1- 2005 101 932

## Beschreibung

### TECHNISCHES GEBIET

Die vorliegende Erfindung betrifft tragbare, medizinische Vorrichtungen, welche einen auf Silikon basierenden Klebestoff zur Befestigung auf der Haut eines menschlichen Körpers umfassen.

### STAND DER TECHNIK

Heutige tragbare medizinische Vorrichtungen wie z.B. Infusionsset zur subkutanen Verabreichung von flüssigen Medikamenten, weisen einen auf Acryl basierenden Klebestoff auf, mit welchem die Vorrichtungen auf der Haut eines Patienten befestigt werden. Beim dauerhaften Einsatz dieser Klebstoffe können diese zu Haütallergien bei Patienten führen, so dass in schwerwiegenden Fällen die Therapie abgebrochen werden muss.

Auch ist es z.B. aus den Dokumenten WO 97/41917 A1, US 2005/0101932 A1, EP 0 780 138 A1, EP 1 213 037 A1, WO 99/58190 A1 und WO 93/09713 A1 bekannt, zur lösbaren Befestigung medizinischer Vorrichtungen auf der Haut von Patienten einen auf Silikon basierenden Klebstoff zu verwenden, welche eine bessere Hautverträglichkeit aufweisen als die Acryl-basierten Klebstoffe. Konkret kommen hier Silikon Pressure Sensitive Adhesives (PSA) zum Einsatz.

### DARSTELLUNG DER-ERFINDUNG

Es ist die Aufgabe der vorliegenden Erfindung, weitere tragbare, medizinische vorrichtungen zur Verfügung zu stellen, welche auch über längere Zeit von Patienten getragen werden können, ohne Hautallergien hervorzurufen.

Diese Aufgabe wird durch den Gegenstand des Anspruchs 1 gelöst. Bevorzugte Gegenstände der Erfindung werden in den abhängigen Ansprüchen beschrieben.

Die erfindungsgemäss tragbare, medizinische Vorrichtung zur Platzierung auf der Haut eines Patienten umfasst ein Silikon Soft Skin Adhesive (SSA) zur lösbaren Befestigung der Vorrichtung auf der Haut.

In einer bevorzugten Ausführungsform handelt es sich um eine Vorrichtung zur subkutanen Verabreichung eines flüssigen Medikaments, bevorzugter um ein Infusionsset zur subkutanen Verabreichung von Insulin. Infusionssets zur subkutanen Verabreichung von flüssigen Medikamenten wie z.B. Insulin, welche erfindungsgemäss mittels eines Silikon Soft Skin Adhesives (SSA) auf der Haut eines Patienten lösbar befestigt werden können, sind z.B. in der EP 0 956 879 und der WO 2004/026375 offenbart. Bevorzugt handelt es sich bei der medizinischen Vorrichtung um eine Einweginfusionspumpe, welche nach Leerung des darin enthaltene Medikamentenreservoirs durch den Patienten von der Hautoberfläche entfernt und vernichtet wird. Die auf Silikon basierenden Klebstoffe eignen sich auch zur Befestigung von medizinischen Vorrichtungen, welche einen implantierbaren Sensor zur Messung eines physiologischen Parameters, wie z.B. des Blutzuckerwertes oder des interstitiellen Glucosewertes, umfassen.

Die erfindungsgemässen Vorrichtungen, wie z.B. Infusionssets bzw. Infusionspumpen, zeichnen sich dadurch aus, dass sie eine gute Hautverträglichkeit aufweisen (hohe Biokompatibilität) und somit bei langdauernden Therapien ohne Allergien hervorzurufen eingesetzt werden können.

In einer weiteren bevorzugten Ausführungsform handelt es sich bei der medizinischen Vorrichtung um eine Infusionspumpe, insbesondere eine Insulinpumpe. Infusionspumpen, welche erfindungsgemäss mittels eines Silikon Soft Skin Adhesives (SSA) auf der Haut eines Menschen lösbar befestigt werden können, sind z.B. in US20040059316 und US6740059 beschrieben.

Bevorzugt ist der Silikon Soft Skin Adhesives (SSA) Teil eines Pflasters, welches auf einer Unterseite der medizinischen Vorrichtung angebracht ist. Das Pflaster erstreckt sich bevorzugt über die gesamte Unterseite des Infusionssets, um eine gute Haftung auf der Haut zu vergeben. Das Pflaster kann auch nur eine Teilfläche der Unterseite des Infusionssets abdecken oder punktförmig auf der Unterseite angebracht sein.

Silikon-Adhesives sind nicht-sensibilisierende Klebstoffe, d.h. Klebstoffe, welche eine gute Biokompatibilität aufweisen. Es gibt zwei Klassen, Silikon pressure-sensitive Adhäsive (PSA) und die Silikon soft skin Adhäsive (SSA). Die Silikon-Adhesives zeichnen sich dadurch aus, dass die Adhäsion bei Feuchtigkeit, wie z.B. Schweiss, und auch bei verschiedenen Temperaturen erhalten bleibt. Zusätzlich weisen die Silikon-Adhesives eine gute Feuchtigkeits- und Luftdurchlässigkeit sowie eine lange Tragedauer auf. Aufgrund dieser Eigenschaften eignen sich die Silikon-Adhesives für die Befestigung medizinischer Vorrichtungen auf der Haut eines Patienten.

### KURZE BESCBREXBUNG DER ZEICHNUNGEN

Weitere Ausgestaltungen, Vorteile und Anwendungen der Erfindung ergeben sich aus den abhängigen Ansprüchen und aus der nun folgenden Beschreibung anhand der Figuren. Dabei zeigen:
Fig. 1 die Struktur von Silikon pressuresehsitiven Adhäsiven;
Fig. 2 die Struktur von Silikon Soft Skin Adhäsiven (SSA); und
Fig. 3 ein erfindungsgemässes Infusionsset.

### WEGE ZUR AUSFÜHRUNG DER ERFINDONG

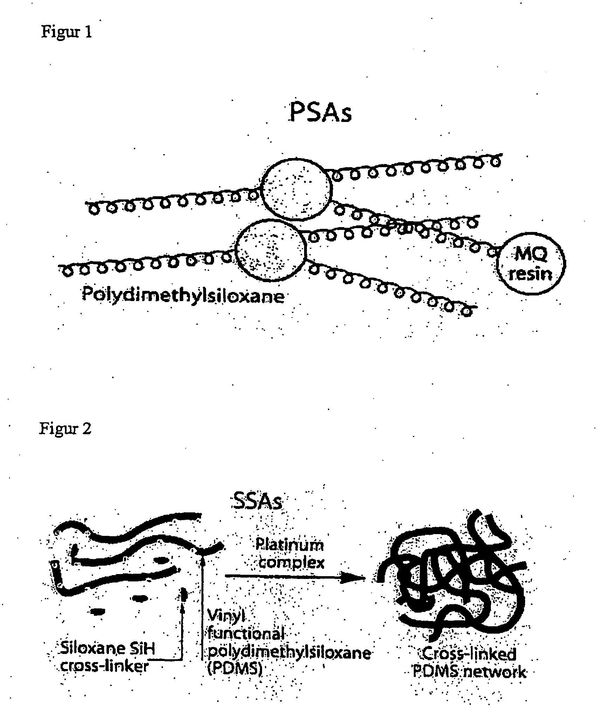

Die Figur 1 zeigt die Struktur von Silikon pressure-sensitiven Adhäsiven. Diese Klebstoffe basieren auf dem Resin-in-Polymer-Prinzip, welches die Kondensation von Dimethiconol zu MQ Resin beinhaltet. Dimethiconol ist ein tief bis mittel visköses Silanol-Polydimethylsiloxane (PDMS). MQ Resin ist ein lösliches, dreidimensionales Silikat Netzwerk aus zwei verschiedenen Untereinheiten, welche beide Silicium enthalten. Das Dimethiconol ist die visköse Komponente des Klebstoffes, und ist verantwortlich für das viskös-elastische Verhalten und beeinflusst die Feuchtigkeits- und Auftragbarkeit des Klebstoffes. Das Resin wirkt als stabilisierendes Element, und ist ebenfalls verantwortlich bei der Elastizität und der Verstärkung der Klebekraft. Silikon PSA können in Heptan, Ethyl Acetat oder volatilen Silikon-Flüssigkeiten gelöst sein.

Die Figur 2 zeigt die Struktur von Silikon Soft Skin Adhesiven (SSA). SSA bestehen aus zwei Teilen und enthalten kein Lösungsmittel. Sie basieren auf einer verkreuzten, elastomeren Struktur. Das Vernetzen ist das Resultat einer Reaktion zwischen Vinyl-funktionalem Polydimethylsiloxane (PDMS) und hydrogen-funktionalem Siloxan. Der Prozess kann durch einen Platinum-Komplex katalysiert werden und es entsteht ein querverbundenes Polymer. Durch ihre andersartige Struktur sind sie eher gelartig. Sie haben etwas andere Eigenschaften als PSA, indem sie weniger elastisch sind, leicht flüchtig sind, und stärker feuchtende Eigenschaften haben. Die Viskosität ist sehr tief, die Ablöseeigenschaften sind sehr ausgeprägt.

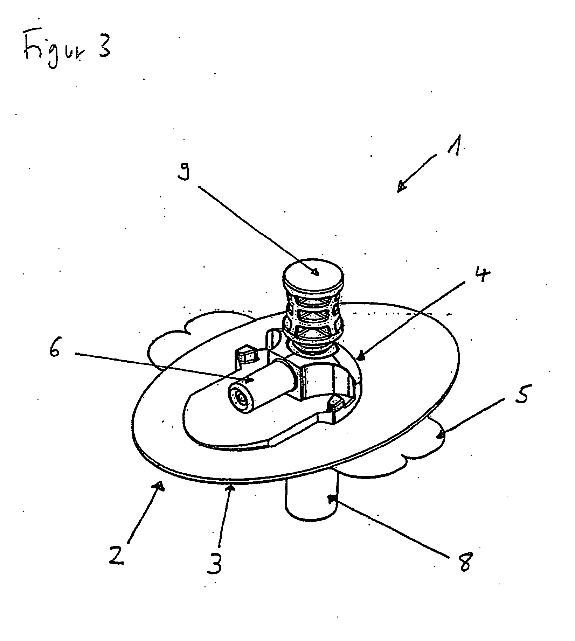

Die Figur 3 zeigt ein erfindüngsgemässes Infusionsset zur Verabreichung eines flüssigen Wirkstoffs in das subkutane Gewebe eines Patienten vor Applikation auf der Haut eines Patienten. Das Infusionsset 1 weißt auf seiner Unterseite 2 ein Pflaster 3 auf, welches den auf Silikon basierenden Klebstoff zur Befestigung des Sets auf der Haut eines Patienten umfasst. Das Pflaster 3 ist vor Gebrauch durch eine Folie abgedeckt, welche einen Griffteil 5 zur leichteren Entfernung der Folie umfasst. Auf der Oberseite des Infusionsset 1 ist ein Hub 4 sichtbar, über welchen die Verbindung mit z.B. einer externen Infusionspumpe hergestellt wird. Die Verbindung des Hub 4 mit der Infusionspumpe kann durch eine lösbare Kopplung des Hub 4 mit einem Schlauch, welcher mit der Pumpe verbunden ist, erfolgen. Am Ende des Schlauchs, welches mit dem Hub 4 verbunden wird, befindet sich ein Verbindungsstück, welches eine lösbare Kopplung mit dem Hub 4 erlaubt. Bei der lösbaren Kopplung handelt es sich bevorzugt um eine Rastverbindung. Die Kopplung des Schlauchs geschieht über den zylindrischen Fortsatz 6 des Hub 4. Das Innere des zylindrischen Fortsatzes bildet einen Fluidkanal, durch welchen die abzugebende Flüssigkeit in den Körper eines Patienten geführt wird. Das Infusionsset 1 umfasst auf der Unterseite eine Kanüle, welche in das subkutane Gewebe eines Patienten eingeführt wird. Die Kanüle ist zumindest teilweise als Hohlnadel ausgestaltet, um einen Flüssigkeitsfluss zu erlauben. Bevorzugt handelt es sich bei der Kanüle um eine weiche Kunststoffkanüle. Vor der Applikation ist die Kanüle durch einen Nadelschutz 8 geschützt. Falls es sich bei der Kanüle um eine weiche Kunststoffkanüle handelt, wird diese bevorzugt mittels einer Metallnadel in die Haut eingebracht. Die Metallnadel weist einen Griff 9 auf, welcher es dem Patienten ermöglicht, die Metallnadel nach Applikation des Sets zu entfernen.

Die Applikation des Infusionssets 1 auf der Haut umfasst folgende Schritte: Der Patient entfernt die Folie des Pflasters 3 und den Nadelschutz 8 und durchsticht mit der Metallnadel, welche den Griff 9 aufweist, die Haut und platziert das Infusionsset auf der Haut. Danach zieht der Patient die Metallnadel mittels des Griffs 9 aus dem Set und schliesst das Infusionsset über eine Kopplung eines Schlauchs an eine Infusionspumpe an. Das Set ist nun zur Verabreichung von flüssigen Wirkstoffen bereit.

## Patentansprüche

1. Tragbare, medizinische Vorrichtung zur Platzierung auf der Haut eines Patienten umfassend einen auf Silikon/basierenden Klebstoff zur lösbaren Befestigung der Vorrichtung auf der Haut, **dadurch gekennzeichnet, dass** der auf Silikon basierende Klebstoff ein Silikon soft skin Adhäsive (SSA) ist.

2. Medizinische Vorrichtung gemäss Anspruch 1, **dadurch gekennzeichnet, dass** es sich um eine Vorrichtung zur subkutanen Verabreichung eines flüssigen Medikaments handelt.

3. Medizinische Vorrichtung gemäss Anspruch 2, **dadurch gekennzeichnet, dass** es sich um eine Infusionspumpe, insbesondere eine tragbare Insulinpumpe, handelt.

4. Medizinische Vorrichtung gemäss Anspruch 2, **dadurch gekennzeichnet, dass** es sich um ein Infusionsset zur subkutanen Verabreichung von Insulin handelt.

5. Medizinische Vorrichtung gemäss Anspruch 1, **dadurch gekennzeichnet, dass** es sich um eine Vorrichtung mit einem Sensor zur Messung eines physiologischen Parameters handelt.

6. Medizinische Vorrichtung gemäss Anspruch 5, **dadurch gekennzeichnet, dass** es sich um einen Sensor zur Messung des Blutzuckerwerts oder des interstitiellen Zuckergehaltes handelt, insbesondere einen elektrochemischen Sensor.

7. Medizinische Vorrichtung gemäss einem der vorherigen Ansprüche, **dadurch gekennzeichnet, dass** der Klebestoff Teil eines Pflasters ist, welches auf einer Oberfläche der medizinischen Vorrichtung, die für den Kontakt mit der Haut eines Patienten ausgestaltet ist, angebracht ist.

8. Medizinische Vorrichtung gemäss Anspruch 7, **dadurch gekennzeichnet, dass** es sich bei der Oberfläche um eine Unterseite der Vorrichtung handelt.

9. Medizinische Vorrichtung gemäss dem Anspruch 7 oder 8, **dadurch gekennzeichnet, dass** die Oberfläche im Wesentlichen flach ist.

## Claims

1. Portable medical device for placement on the skin of a patient including a silicone-based adhesive for releasable fixing of the device on the skin, **characterized in that** the silicone-based adhesive is a silicone soft skin adhesive (SSA).

2. Medical device according to claim 1 **characterized in that** it is a device for the subcutaneous administration of a liquid medicine.

3. Medical device according to claim 2 **characterised in that** it is an infusion pump, in particular a portable insulin pump.

4. Medical device according to claim 2 **characterised in that** it is an infusion set for the subcutaneous administration of insulin.

5. Medical device according to claim 1 **characterised in that** it is a device having a sensor for measuring a physiological parameter.

6. Medical device according to claim 5 **characterised in that** it involves a sensor for measuring the blood sugar value or the interstitial sugar content, in particular an electrochemical sensor.

7. Medical device according to one of the preceding claims **characterised in that** the adhesive is part of a plaster which is disposed on a surface of the medical device, which is designed for contact with the skin of a patient.

8. Medical device according to claim 7 **characterised in that** the surface is an underside of the device.

9. Medical device according to claim 7 or claim 8 **characterised in that** the surface is substantially flat.

## Revendications

1. Dispositif médical portable destiné à être placé sur la peau d'un patient, comprenant un adhérent à base de silicone pour fixation amovible du dispositif sur la peau, **caractérisé en ce que** l'adhérent à base de silicone est un adhésif soft skin (SSA) silicone.

2. Dispositif médical selon la revendication 1, **caractérisé en ce qu'**il s'agit d'un dispositif d'administration sous-cutanée d'un médicament liquide.

3. Dispositif médical selon la revendication 2, **caractérisé en ce qu'**il s'agit d'une pompe à perfusion, en particulier d'une pompe à insuline portable.

4. Dispositif médical selon la revendication 2, **caractérisé en ce qu'**il s'agit d'un kit de perfusion pour l'administration sous-cutanée d'insuline.

5. Dispositif médical selon la revendication 1, **caractérisé en ce qu'**il s'agit d'un dispositif comportant un capteur destiné à mesurer un paramètre physiologique.

6. Dispositif médical selon la revendication 5, **caractérisé en ce qu'**il s'agit d'un capteur servant à mesurer la glycémie ou le taux de sucre interstitiel, en particulier un capteur électrochimique.

7. Dispositif médical selon l'une des revendications précédentes, **caractérisé en ce que** l'adhérent fait partie d'un pansement qui est appliqué sur une surface du dispositif médical qui est conçu pour le contact avec la peau d'un patient.

8. Dispositif selon la revendication 7, **caractérisé en ce que** la surface est une face inférieure du dispositif.

9. Dispositif médical selon la revendication 7 ou 8, **caractérisé en ce que** la surface est essentiellement plane.
